# EUROPÄISCHE PATENTSCHRIFT

(11) **EP 2 123 324 B1**
(45) Veröffentlichungstag und Bekanntmachung des Hinweises auf die Patenterteilung: **07.01.2015**
(21) Anmeldenummer: 09158446.6
(22) Anmeldetag: 22.04.2009
(51) Int. Cl.: A61N 1/37

(54) **EKG-Analysevorrichtung**
ECG Analyzing Device
Appareil de commande de direction

(30) Priorität: 20.05.2008 DE 102008024453
(43) Veröffentlichungstag der Anmeldung: 25.11.2009
(73) Patentinhaber: Biotronik CRM Patent AG, 6341 Baar (CH)
(72) Erfinder: Diem, Björn Henrik, 12161, Berlin (DE); Kraus, Alexander, 10407, Berlin (DE); Anosov, Oleg, 91054, Erlangen (DE)
(74) Vertreter: Lindner-Vogt, Karin L.

(56) Entgegenhaltungen:
- US-B1- 6 589 187

## Beschreibung

Die Erfindung betrifft eine Vorrichtung zur Verarbeitung von Elektrokardiogrammsignalen und Markersignalen. Insbesondere betrifft die Erfindung eine Vorrichtung, die intrakardial, beispielsweise mit Hilfe eines medizinischen Implantats, wie einem Herzschrittmacher oder einem implantierbaren Cardioverter-Defibrillator (ICD), gewonnen wurden. Derartige Implantate generieren üblicherweise auch Markersignale.

Zum Empfangen solcher Elektrokardiogrammsignale und Markersignale besitzt die Vorrichtung eine Eingangsschnittstelle. Mit dieser Eingangsschnittstelle ist eine Analyseeinheit verbunden, die die Elektrokardiogrammsignale und Markersignale verarbeitet. Mit der Analyseeinheit wiederum verbunden ist eine Ausgabeschnittstelle zur Ausgabe von Daten, die einen Analysebericht repräsentieren und geeignet sind, durch ein Wiedergabegerät, beispielsweise einen Bildschirm oder Drucker, dargestellt zu werden.

Wie bereits erwähnt sind Implantate, wie implantierbare Herzschrittmacher oder ICD, die intrakardiale Elektrokardiogramme aufnehmen und Markersignale gewinnen, grundsätzlich bekannt. Die intrakardialen Elektrokardiogramme (IEGM) liegen dabei typischerweise in Form einer Zeitreihe von Werten vor, die ein zeitabgetastetes, ursprünglich analoges Signal repräsentieren. Ein derartiges Elektrokardiogramm besitzt verschiedene typische und periodisch wiederkehrende Signalmerkmale, die solche kardialen Ereignisse repräsentieren, wie die Kontraktionen eines rechten oder linken Ventrikels oder die Kontraktionen des rechten oder linken Atriums. Typischerweise werden die Kontraktionen des rechten oder linken Atriums repräsentierenden Signalmerkmale als P-Wellen bezeichnet, während die mit der Kontraktion der Ventrikel einhergehenden Signalmerkmale sich in einer sogenannten R-Zacke widerspiegeln. Das Implantat erstellt auf Basis eines derartigen Elektrokardiogramms Markersignale, die das zeitliche Auftreten detektierter kardialer Ereignisse markieren, dadurch, dass das Implantat P-Wellen und R-Zacken detektiert, beispielsweise durch entsprechenden Schwellwertvergleich. Hierzu wird typischerweise ein intraatriales Kardiogramm einer atrialen Sensingeinheit des Implantats zugeführt, die dazu aufgenommene intraatriale Elektrokardiogrammsignale ständig mit einem Schwellwert vergleicht und bei Schwellwertüberschreitung ein Markersignal generiert, das ein atriales Ereignis, d.h. eine Kontraktion des Atriums, widerspiegelt. Entsprechend wird regelmäßig ein intraventrikuläres Elektrokardiogramm durch eine ventrikuläre Sensingeinheit mit einem Schwellwert verglichen und bei Überschreitung dieses Schwellwertes ein ventrikuläres Markersignal erzeugt, das die Kontraktion des entsprechenden Ventrikels markiert. Weitere typische Markersignale enthalten Informationen über die Bewertung und Reaktion (z.B. Therapie) des Implantats. Dies ist zum Beispiel in US6589187 beschrieben.

Hintergrund ist, dass die Kontraktion eines Atriums oder eines Ventrikels mit einer Depolarisation des jeweiligen Herzmuskelgewebes (Myokards) einhergeht, die zu erfassbaren Potenzialen führt, welche durch intraatrial oder intraventrikulär angeordnete Elektroden aufzunehmen sind und im Ergebnis zu den entsprechenden Signalen im intraatrialen und im intraventrikulären Elektrogramm führen.

Über eine entsprechende Schnittstelle zur drahtlosen Datenübertragung sind solche Implantate in der Lage, sowohl aufgenommene Elektrogramme in Form von Elektrokardiogrammsignalen als auch aus den Elektrokardiogrammen gewonnene Markersignale telemetrisch auf ein externes Gerät zu übertragen.

Die Erfindung betrifft eine Vorrichtung zur Auswertung der so gewonnenen Signale. Die Vorrichtung ist dabei vorzugsweise außerhalb des Implantats angeordnet, kann jedoch im Prinzip auch Teil eines jeweiligen Implantats sein.

In einem typischen Szenario ist die hier gemeinte Vorrichtung Bestandteil eines Servicecenters, das typischerweise über ein in der Nähe eines jeweiligen Patienten befindlichen und als Realisation dienenden externen Gerätes in der Lage ist, von einem Implantat Elektrokardiogrammsignale, Marker- und Implantatsdaten zu empfangen und zu verarbeiten. Ein derartiges zentrales Servicecenter stellt ohne weiteres die zur Verarbeitung erforderlichen Ressourcen zur Verfügung, so dass diese nicht im Implantat vorgehalten werden müssen. Mit fortschreitender Technik ist es auch denkbar, die Vorrichtung in ein Implantat zu integrieren.

Aufgabe der Erfindung ist es, eine Vorrichtung der eingangs genannten Art zu schaffen, die einen Arzt bei der Versorgung eines Patienten unterstützt. Solche Vorrichtungen sind grundsätzlich bekannt. Mit der hier betroffenen Erfindung wird angestrebt, die Arbeit des Arztes weiter zu erleichtern, um auf diese Weise auch die Betreuung des Patienten ggf. zu verbessern.

Erfindungsgemäß wird das angestrebte Ziel durch eine Vorrichtung gemäß Anspruch 1 erreicht.

Vorzugsweise sind die zu detektierenden Auffälligkeiten erfassungstechnischer Natur, beispielsweise Auffälligkeiten, die auf einen Elektrodenbruch hindeuten, Auffälligkeiten, die auf ein Oversensing hindeuten, d.h. darauf hindeuten, dass in einem vorgegebenen Elektrokardiogramm zu viele Ereignisse detektiert werden, Auffälligkeiten, die auf eine Fernfeldwahrnehmung von Ereignissen in einer anderen Herzkammer hindeuten oder ähnliches. Indem die erfindungsgemäße Vorrichtung derartige Auffälligkeiten automatisch detektiert und einen für den Arzt leicht verständlichen Analysebericht generiert, erleichtert die Vorrichtung es dem Arzt, mögliche Störungen rechtzeitig wahrzunehmen und entsprechende Gegenmaßnahmen zu ergreifen. Da der Analysebericht erfindungsgemäß immer einen jeweiligen, auch die Auffälligkeiten repräsentierenden Elektrokardiogrammsignalabschnitt und/oder Markersignalabschnitt in graphischer Wiedergabe enthält, kann sich der Arzt aufgrund der zugrunde liegenden Signale selbst ein Bild von der Auffälligkeit machen. Da die auffälligen Signalabschnitte in dem Analysebericht grafisch hervorgehoben sind, weiß der Arzt auf den ersten Blick, welche Signalabschnitte er ggf. selbst noch einmal unter die Lupe nehmen muss. Da der Analysebericht außerdem eine Benennung der jeweiligen Auffälligkeit enthält, weiß der Arzt auch, welche tatsächliche oder vermeintliche Auffälligkeit die Vorrichtung automatisch detektiert hat.

Vorzugsweise ist die Vorrichtung durch einen Arzt konfigurierbar, indem die Vorrichtung eine Eingabeschnittstelle aufweist, die mit der Analyseeinheit verbunden und in Verbindung mit dieser ausgebildet ist, Benutzervorgaben entgegenzunehmen, welche die Arbeitsweise der Analyseeinheit bei der Verarbeitung der Elektrokardiogrammsignale und Markersignale beeinflussen. Solche Benutzervorgaben können beispielsweise darin bestehen, dass der Arzt aus einem Katalog von Auffälligkeiten, die die Vorrichtung grundsätzlich detektieren kann, diejenigen Auffälligkeiten auswählt, deren Detektion der Arzt wünscht. Die Vorrichtung und insbesondere die Analyseeinheit ist für diesen Fall so ausgebildet, dass sie bestimmte, der Auswahl entsprechende Untereinheiten, also Teilmodule der Analyseeinheit, aktiviert oder deaktiviert. Falls für die Detektion einer jeweiligen Auffälligkeit bestimmte Kriterien gelten sollen, können diese durch entsprechende Benutzervorgaben durch den Arzt eingestellt werden.

Wie bereits angedeutet ist die Analyseeinheit vorzugsweise dazu ausgebildet, verschiedene Arten von Auffälligkeiten unabhängig voneinander zu detektieren und weist entsprechende Untereinheiten bzw. Teilmodule auf.

In diesem Zusammenhang ist eine Analyseeinheit vorteilhaft, die einen Rauschdetektor als Untereinheit aufweist, die ausgebildet ist, in einem Elektrokardiogrammsignal Abschnitte zu detektieren, die über ein vorgegebenes Maß hinaus verrauscht sind. Der Rauschdetektor erkennt somit starkes Rauschen in den Elektrokardiogrammsignalen. Die Schwelle, ab der solches Rauschen erkannt wird, kann dabei vom Arzt über die Eingabeschnittstelle einzustellen sein.

Zusätzlich oder alternativ kann die Analyseeinheit einen Artefaktdetektor als Untereinheit aufweisen, der ausgebildet ist, in einem Elektrokardiogrammsignal Signalabschnitte zu detektieren, die Artefakte, beispielsweise Polarisationsartefakte, enthalten. Polarisationsartefakte können im Anschluss an die Abgabe eines Stimulationsimpulses oder Defibrillationsschocks im Elektrokardiogrammsignal aufgezeichnet werden. Ein solcher Impuls oder Schock hat die Folge, dass eine Grenzschicht um eine signalaufnehmende Elektrode polarisiert wird, so dass die Elektrode im Ergebnis dadurch Potenziale aufnimmt, die jedoch keine auf eine Depolarisation des Myokards zurückgehenden Potenziale sind, sondern Artefakte.

Ebenfalls kann die Analyseeinheit eine Oversensing-Klassifikationseinheit als Untereinheit aufweisen, die ausgebildet ist, in einem Elektrokardiogramm Signalabschnitte zu detektieren, die Signalmerkmale aufweisen, die auf Oversensing hinweisen.

Hierzu weist die Analyseeinheit vorzugsweise einen eigenen Ereignisdetektor als Untereinheit auf, der in den empfangenen Elektrokardiogrammsignalen Ereignisse, wie atriale Depolarisationen oder ventrikuläre Depolarisationen, detektiert. Vorzugsweise ist der Ereignisdetektor dazu ausgebildet, separat vorliegende Elektrokardiogrammsignale für das Atrium und den Ventrikel in Verbindung miteinander zu verarbeiten, um beispielsweise bei der Detektion von atrialen Ereignissen in einem interatrialen Elektrokardiogrammsignal solche Signalauslenkungen erkennen zu können, die möglicherweise auf die Fernfeldwahrnehmung ventrikulärer Ereignisse zurückgehen. Dabei kann der jeweilige Ereignisdetektor grundsätzlich so ausgebildet sein wie eine implantatinterne Sensingeinheit, d.h. Ereignisse werden durch Vergleich mit einem jeweils angemessenen Schwellwert detektiert. Hierfür kann der Ereignisdetektor in der erfindungsgemäßen Vorrichtung jedoch aufwändiger gestaltet sein als eine Sensingeinheit in einem Implantat.

Beispielsweise kann der Ereignisdetektor auch eine Morphologieklassifikationseinheit aufweisen oder mit einer solchen verbunden sein, die Signalabschnitte, die für eine Polarisation des jeweiligen Myokards charakteristisch sind, durch Signalformanalyse detektiert, d.h. nicht durch einfachen Schwellwertvergleich, sondern beispielsweise durch weitergehende Analyse, wie Reihenfolge und Polarität der Auslenkungen etc.

Wenn die Analyseeinheit der erfindungsgemäßen Vorrichtung auf diese Weise selbsttätig atriale und ventrikuläre Ereignisse detektiert hat, kann sie diese durch die Oversensing-Klassifikationseinheit mit empfangenen Markersignalen vergleichen. Wenn sich dabei herausstellt, dass die Analyseeinheit der erfindungsgemäßen Vorrichtung für einen Signalabschnitt wesentlich weniger Ereignisse detektiert hat als es entsprechende Markerereignisse in dem entsprechenden Markersignal gibt, bedeutet dies, dass das die Markersignale generierende Implantat möglicherweise zu viele vermeintliche Ereignisse detektiert hat. Dies bedeutet, es liegt ein Oversensing vor, weil beispielsweise die Detektionsschwelle für die jeweilige Sensingeinheit des Implantats nicht optimal eingestellt ist.

Unabhängig von der Oversensing-Klassifikationseinheit - aber auch in Verbindung mit dieser - ist es vorteilhaft, wenn die Analyseeinheit einen Markerkorrelator als Untereinheit aufweist, der ausgebildet ist, zeitliche Korrelationen zwischen von der Analyseeinheit detektierten kardialen Ereignissen und entsprechenden Markern in den jeweiligen Markersignalen zu bestimmen. Wenn diese Korrelationsanalyse eine hohe Übereinstimmung ergibt, ist es ein Zeichen dafür, dass kein Oversensing vorliegt. Eine stärkere Abweichung der empfangenen Markersignale von den von der Analyseeinheit detektierten Ereignissen hingegen ist ein Hinweis auf ein mögliches Over- oder Undersensing, d.h. beispielsweise auf eine falsch angepasste Detektionsschwelle einer Sensingeinheit des Implantats.

Vorzugsweise weist die Vorrichtung außerdem eine Bewertungseinheit als Untereinheit auf, die mit der Analyseeinheit verbunden oder deren Bestandteil ist und ausgebildet ist, von der Analyseeinheit detektierte Auffälligkeiten anhand vorgegebener Kriterien hinsichtlich ihrer Bedeutung derart zu bewerten, dass für den Patienten kritische Auffälligkeiten eine andere Bewertung erfahren als für den Patienten unkritische Auffälligkeiten. Beispielsweise kann die Bewertung dreiwertig sein, d.h. zwischen besonders kritischen Auffälligkeiten, durchschnittlich kritischen Auffälligkeiten und weniger kritischen Auffälligkeiten unterscheiden.

Vorzugsweise ist die Analyseeinheit in diesem Zusammenhang dazu ausgebildet, einen jeweiligen Analysebericht auf eine von der Bewertungseinheit generierte Bewertung hinzuzufügen, also im Klartext beispielsweise die Bemerkungen "sehr kritisch", "weniger kritisch" oder "unkritisch" hinzuzufügen. Die Bewertungen können aber auch in grafischer Form dem Analysebericht zugefügt werden, beispielsweise indem Felder des Analyseberichts in einer Farbe, beispielsweise Rot für besonders kritische Auffälligkeiten, Gelb für mittelkritische Auffälligkeiten und Weiß bei Fehlen kritischer und mittelkritischer Auffälligkeiten, hinzugefügt werden.

Weiterhin ist es vorteilhaft, wenn die Vorrichtung eine Empfehlungseinheit als Untereinheit aufweist, die mit der Analyseeinheit verbunden ist und ausgebildet ist, auf der Basis von der Analyseeinheit detektierten Auffälligkeiten eine Handlungsempfehlung für den Arzt zu generieren. Im Falle eines Over- oder Undersensing könnte die Handlungsempfehlung beispielsweise dahin gehen, die Detektionsschwelle der jeweiligen Sensingeinheit zu erhöhen bzw. herabzusetzen. Im Falle der Detektion eines Elektrodenbruchs könnte die Empfehlung lauten, die Stimulationselektrode zu ersetzen.

Zum Erzeugen eines jeweiligen Analyseberichts aus den zuvor von der Analyseeinheit gewonnenen Daten ist vorzugsweise ein ggf. konfigurierbarer Berichtsgenerator vorgesehen.
Vorzugsweise wird die Auflistung der detektierten Auffälligkeiten durch eine Priorisierung und/oder eine Wiedergabe eines Elektrokardiogrammsignal- und/oder eines Markersignalabschnitts in graphischer Wiedergabe ggf. mit Markierung der die Auffälligkeit repräsentierenden Signalabschnitte und eine Benennung der Auffälligkeit ergänzt.

Weitere bevorzugte Ausführungsvarianten ergeben sich aus verschiedenen Kombinationen der hier beschriebenen Ausführungsvarianten und aus der nachfolgenden Beschreibung eines Ausführungsbeispiels.

Im Folgenden soll nun die Erfindung anhand eines solchen Ausführungsbeispiels mit Bezug auf die Figuren näher erläutert werden. Von den Figuren zeigt:
- Fig. 1:: Einen Überblick über ein System zur Patientenversorgung mit einer erfindungsgemäßen Herzanalysevorrichtung;
- Fig. 2:: ein alternatives System mit einer erfindungsgemäßen ElektrokardiogrammAnalysevorrichtung gemäß der Erfindung;
- Fig. 3:: eine schematische Darstellung einer erfindungsgemäßen Elektrokardiogramm-Analysevorrichtung;
- Fig. 4:: eine Darstellung der Analyseeinheit der erfindungsgemäßen Elektrokardiogramm-Analysevorrichtung;
- Fig. 5:: ein Beispiel für eine IEGM Präsentation in dem von der erfindungsgemäßen Elektrokardiogramm-Analysevorrichtung gemäß Fig. 3 erzeugten Analysebericht;
- Fig. 6:: eine zweite IEGM Präsentation in einem von der erfindungsgemäßen Elektrokardiogramm-Analysevorrichtung gemäß Fig. 3 erzeugten anderen Analysebericht;
- Fig. 7:: eine dritte IEGM Präsentation in einem weiteren von der erfindungsgemäßen Elektrokardiogramm-Analysevorrichtung gemäß Fig. 3 erzeugten Analysebericht; und
- Fig. 8:: ein viertes Beispiel einer IEGM Präsentation in einem weiteren von der erfindungsgemäßen Elektrokardiogramm-Analysevorrichtung gemäß Fig. 3 erzeugten Analysebericht.

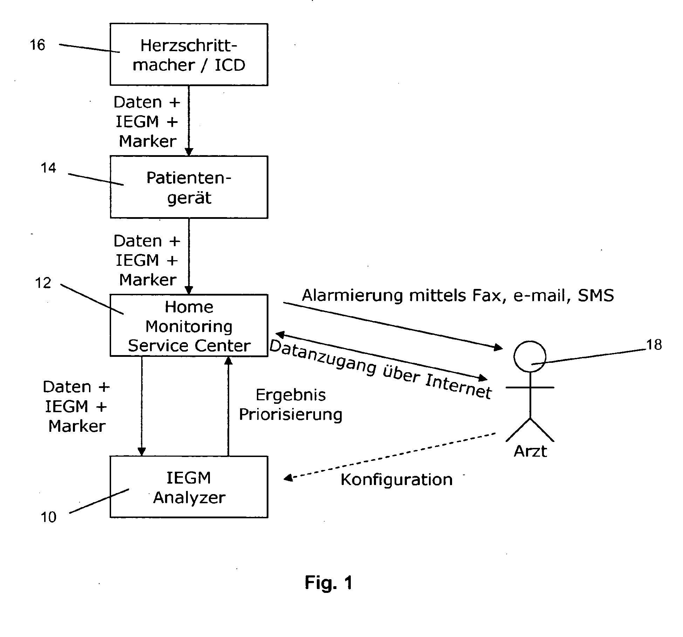

Das in Fig. 1 abgebildete System besitzt eine Elektrokardiogramm-Analysevorrichtung 10 als Bestandteil eines zentralen Servicecenters 12, das über ein Patientengerät 14 zumindest zeitweise mit einem implantierbaren Herzstimulator 16 (Herzschrittmacher und/oder ICD) in Verbindung steht. Auf diese Weise ist das zentrale Servicecenter 12 in der Lage, von dem implantierbaren Herzschrittmacher in an sich bekannter Weise begonnene Elektrokardiogrammsignale und Markersignale zu empfangen. Im Falle eines Zweikammerherzschrittmachers sind dies beispielsweise ein intraatriales Elektrokardiogrammsignal und ein intraventrikuläres Elektrokardiogrammsignal sowie ein Markersignal mit einzelnen Markern für atriale und ventrikuläre Ereignisse, die von entsprechenden Sensingeinheiten des implantierbaren Elektrostimulators 16 durch interne Auswertung der intrakardialen Elektrokardiogramme gewonnen wird. Wie bereits eingangs erläutert geschieht dies typischerweise durch Vergleich eines jeweiligen intrakardialen Elektrokardiogrammsignals mit entsprechenden Detektionsschwellen, wobei die Detektionsschwelle für die Detektion atrialer Ereignisse aus einem intraatrialen Elektrokardiogramm in der Regel eine andere ist als die Detektionsschwelle für die Detektion ventrikulärer Ereignisse in einem intraventrikulären Elektrokardiogramm.

Über das zentrale Servicecenter 12 hat ein Arzt 18 typischerweise Zugriff auf die seitens des implantierbaren Herzstimulators 16 empfangenen Elektrokardiogrammsignale und Markersignale. Dieser Zugang kann beispielsweise über das Internet erfolgen, wobei das zentrale Servicecenter 12 typischerweise ausgebildet ist, den Arzt von sich aus in bestimmten Fällen per Email, Fax oder SMS zu alarmieren.

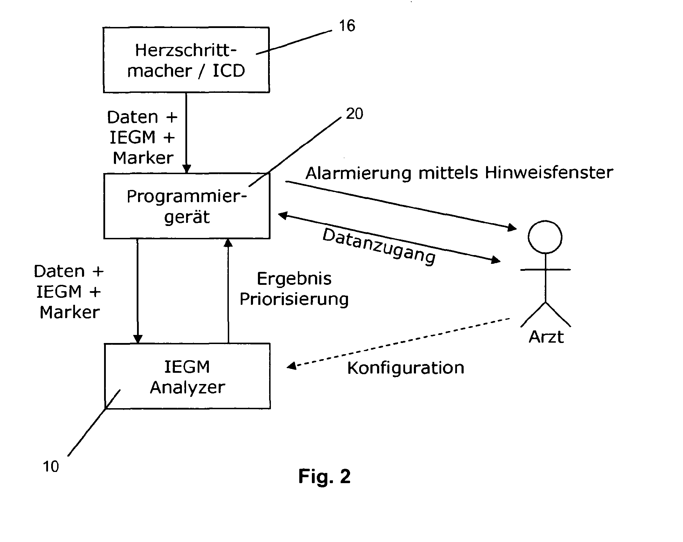

Fig. 2 zeigt, dass die erfindungsgemäße Elektrokardiogrammanalysevorrichtung 10 auch Bestandteil eines Programmiergerätes 20 sein kann, welches ein Arzt bei einer Visite zum Auslesen der in einem implantierbaren Herzstimulator 16 gespeicherten Daten verwendet. Da sich in diesem Falle das Programmiergerät 20 in der Nähe des Herzstimulators 16 befindet, erfolgt die Übertragung der entsprechenden Elektrokardiogrammsignale und Markersignale direkt vom implantierbaren Herzstimulator 16 zum Programmiergerät 20, so dass diese Signale dem Arzt 18 unmittelbar zur Verfügung stehen. Typischerweise besitzt ein Programmiergerät 20 eine grafische Anzeige, die es erlaubt, neben Text auch Elektrokardiogrammsignalabschnitte grafisch wiederzugeben. Darüber hinaus besitzt ein Programmiergerät 20 typischerweise auch eine Eingabeeinheit, die es dem Arzt 18 erlaubt, Eingaben zur Steuerung der Programmiergerätes und insbesondere auch zur Steuerung der Elektrokardiogrammanalysevorrichtung 10 zu machen.

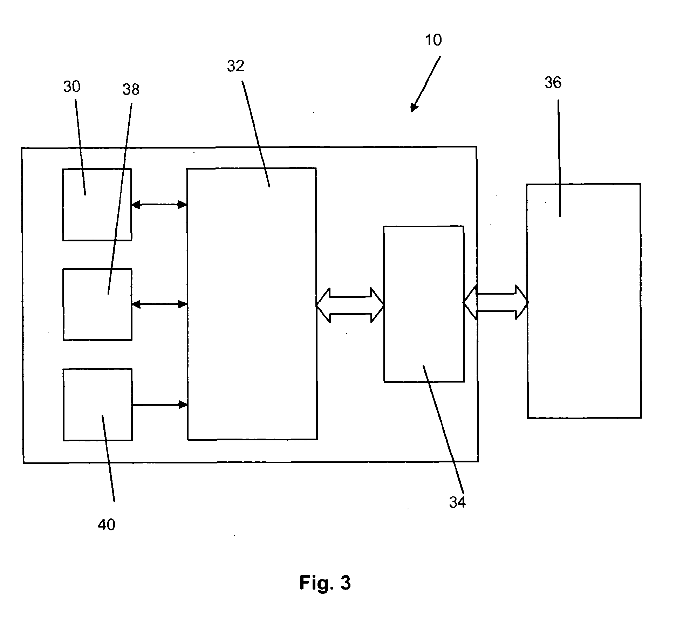

In Fig. 3 ist die Elektrokardiogrammanalysevorrichtung 10 aus Fig. 1 und Fig. 2 detaillierter dargestellt.

Die Vorrichtung 10 besitzt eine Eingangsschnittstelle 30 zum Empfangen von Elektrokardiogrammsignalen und Markersignalen, die von einem Implantat 16 erzeugt sind. Mit der Eingangsschnittstelle 30 ist eine Analyseeinheit 32 verbunden, die zum Verarbeiten der Elektrokardiogrammsignale und Markersignale auf die nachfolgend näher beschriebene Art und Weise ausgebildet ist. Mit der Analyseeinheit 32 ist wiederum eine Ausgangsschnittstelle 34 verbunden, die zur Ausgabe von einen Analysebericht repräsentierenden Daten an ein Wiedergabegerät 36, beispielsweise einen Bildschirm oder einen Drucker, ausgebildet ist. Außerdem ist die Analyseeinheit 32 mit einem Speicher 38 verbunden. Eine Eingabeschnittstelle 42 ist ebenfalls mit der Analyseeinheit 32 verbunden und erlaubt es, die Analyseeinheit mit einem Eingabegerät, wie einer Tastatur oder ähnlichem, zu verbinden, die Bestandteil der Vorrichtung 10 sein kann.

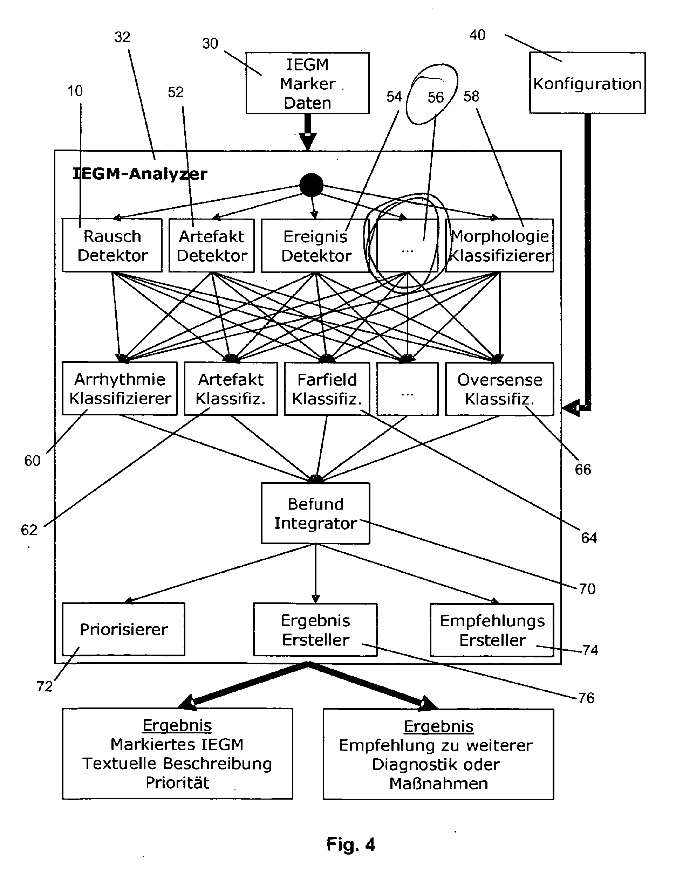

Fig. 4 zeigt einige Bestandteile der Vorrichtung 10 in detaillierterer Darstellung. Insbesondere ist die Analyseeinheit 32 detaillierter dargestellt, die eingangsseitig mit der Eingangsschnittstelle 30 sowie der Eingabeschnittstelle 40 verbunden ist. Über die Eingangsschnittstelle 30 empfängt die Analyseeinheit 32 die zu verarbeitenden Elektrokardiogrammsignale und Markersignale und über die Eingabeschnittstelle 40 können der Analyseeinheit 32 Steuerbefehle zur Konfiguration der Analyseeinheit 32 eingegeben werden.

Die über die Eingangsschnittstelle 30 empfangenen Elektrokardiogrammsignale und Markersignale werden je nach Konfiguration verschiedenen Untereinheiten (oder auch Teilmodulen) der Analyseeinheit 32 zugeführt. Eine erste Kategorie von Untereinheiten betrifft die unmittelbare Signalaufbereitung, während eine zweite Kategorie von Untereinheiten der eigentlichen Detektion von Auffälligkeiten auf Basis der aufbereiteten Signale dient.

Zu den Untereinheiten der ersten Kategorie zählen ein Rauschdetektor 50, ein Artefaktdetektor 52, ein Ereignisdetektor 54, ein Markerkorrelator 56 und ein Morphologieklassifizierer 58.

Anders als in Fig. 4 angedeutet können dabei der Markerkorrelator, der Ereignisdetektor und der Morphologieklassifizierer miteinander verbunden sein, so dass insbesondere der Ereignisdetektor 54 auch Ausgangssignale des Morphologieklassifizierers 58 verarbeiten kann und dass der Markerkorrelator 56 Ausgangssignale des Ereignisdetektors 54 verarbeiten kann.

Der Rauschdetektor 50 ist dazu ausgebildet zu detektieren, wie hoch ein Rauschanteil an einem über die Eingangsschnittstelle 30 empfangenen Elektrokardiogrammsignal ist.

Der Artefaktdetektor 52 ist dazu ausgebildet, mögliche Artefakte im Elektrokardiogrammsignal zu detektieren und kann dazu beispielsweise mit dem Morphologieklassifizierer 58 zusammenwirken.

Der Ereignisdetektor 54 ist dazu ausgebildet, in dem Elektrokardiogrammsignal Ereignisse zu detektieren, d.h. Signale, die auf eine Kontraktion der jeweiligen Herzkammer oder des jeweiligen Vorhofs hindeuten. Wie zuvor angedeutet können auch mehrere Ereignisdetektoren vorgesehen sein, und zwar insbesondere dann, wenn die Analyseeinheit 32 nicht nur ein einziges Elektrokardiogrammsignal empfängt, sondern beispielsweise zwei oder drei Elektrokardiogrammsignale, die separat im rechten Atrium, im rechten Ventrikel und/oder im linken Ventrikel eines Herzens aufgenommen wurden.

Auch ein jeweiliger Ereignisdetektor 54 greift sinnvoller Weise auf Signale zurück, die vom Morphologieklassifizierer 58 generiert wurden. Beispielsweise kann der Morphologieklassifizierer 58 ausgebildet sein, anhand der Signalmorphologie Artefakte, Fernfeldereignisse oder auch echte kardiale Ereignisse in der jeweiligen Herzkammer voneinander zu unterscheiden und zu detektieren.

Der Ereignisdetektor 54 führt im Ergebnis eine selbständige Analyse der empfangenen Elektrokardiogrammsignale durch und erzeugt auf diese Weise in der Analyseeinheit 32 generierte Markersignale, die anschließend mit den über die Eingangsschnittstelle 30 empfangenen Markersignalen verglichen werden kann. Dies geschieht durch den Markerkorrelator 56. Idealerweise stimmen die in der Analyseeinheit 32 generierten Markersignale mit den von der Analyseeinheit 32 empfangenen Markersignalen überein. Dies würde im Ergebnis bedeuten, dass die in der Regel unterschiedlich arbeitenden entsprechenden Sensingeinheiten des Implantats 16 und der Ereignisdetektor oder die Ereignisdetektoren 54 der Analyseeinheit 32 miteinander übereinstimmende Ergebnisse liefern. Falls jedoch die über die Eingangsschnittstelle 30 empfangenen Markersignale wesentlich mehr Ereignismarker enthalten als die von dem Ereignisdetektor 54 erzeugten Markersignale, ist dies ein Hinweis darauf, dass die entsprechende Sensingeinheit des Implantats 16 möglicherweise zu viele Ereignisse detektiert, was beispielsweise eine Folge von Oversensing sein kann. Zum Oversensing kommt es, wenn eine Sensingeinheit des Implantats eine Ereignisdetektion anhand eines Schwellwertevergleichs eines intrakardialen Elektrokardiogramms mit einer Detektionsschwelle durchführt und die Detektionsschwelle so niedrig angesetzt ist, dass nicht nur originär kardiale Ereignisse, sondern auch andere Signalauslenkungen fälschlicherweise als kardiale Ereignisse detektiert werden. Oversensing geht so beispielsweise auf eine falsch gewählte Detektionsschwelle für die jeweilige Sensingeinheit des Implantats zurück.

Umgekehrt kann das über die Eingangsschnittstelle 30 empfangene Markersignal auch wesentlich weniger Ereignismarker enthalten als durch den Ereignisdetektor 54 detektiert werden. Dies kann ein Hinweis auf Undersensing sein, d.h. beispielsweise auf eine zu hoch eingestellte Detektionsschwelle der jeweiligen Sensingeinheit des Implantats 16, so dass nicht alle Ereignisse im jeweiligen intrakardialen Elektrokardiogrammsignal zuverlässig detektiert werden, weil einige zu möglicherweise nicht ausreichend hohen Auslenkungen des Elektrokardiogrammsignals führen.

Eine zweite Kategorie von Untereinheiten bzw. Teilmodulen der Analyseeinheit 32 sind eine Reihe von Klassifikationseinheiten, die jeweils ausgebildet sind, bestimmte Auffälligkeiten in den empfangenen Elektrokardiogrammsignalen oder Markersignalen zu detektieren. Zu diesen Untereinheiten der zweiten Kategorie zählen beispielsweise ein Arrhythmieklassifizierer 60, ein Artefaktklassifizierer 62, ein Farfieldklassifizierer 64 und ein Oversenseklassifizierer 66. Weitere Klassifizierer können ebenfalls vorgesehen sein. Wie in Fig. 4 angedeutet können alle Klassifizierer mit Untereinheiten der ersten Kategorie verknüpft sein, um die Ausgangssignale verschiedener Untereinheiten der ersten Kategorie für die Klassifikation von Auffälligkeiten in dem Elektrokardiogrammsignal oder dem Markersignal heranziehen zu können.

Die so detektierten Auffälligkeiten werden einem Befundintegrator 70 zugeführt, der mit einer Bewertungseinheit 72 und einer Empfehlungseinheit 74 zusammenwirkt sowie mit einem Berichtsgenerator 76.

Die Bewertungseinheit 72 ist dazu ausgebildet, die Ausgangswerte der Untereinheiten der zweiten Kategorie hinsichtlich der Bedeutsamkeit einer möglichen Auffälligkeit zu bewerten. Auffälligkeiten, die auf ernste Störungen hinweisen, erhalten dementsprechend eine Bewertung, die einer hohen Priorität entspricht, während Auffälligkeiten, die auf eine weniger ernste Störung hinweisen, eine Bewertung erfahren, die auf eine mittlere oder eine niedrige Priorität hinauslaufen.

Die Empfehlungseinheit 74 knüpft an jeweils detektierte Auffälligkeiten eine Empfehlung. Beispielsweise kann dies im Falle des Undersensing die Empfehlung sein, die Detektionsschwelle der jeweiligen Sensingeinheit des Implantats 16 herabzusetzen oder im Falle der Detektion eines Elektrodenbruchs die Empfehlung sein, die Elektrodenleitung auszutauschen.

Die Ergebnisse des Befundintegrators 70 sowie der Bewertungseinheit 72 und der Empfehlungseinheit 74 werden schließlich dem Analyseberichtgenerator 76 zugeführt, der zum einen einen Analysebericht generiert, der eine grafische Darstellung des jeweils von einer Auffälligkeit betroffenen Elektrokardiogrammsignalabschnitts oder Markersignalabschnitts enthält sowie darüber hinaus eine Benennung der Auffälligkeit, die die Analyseeinheit 32 detektiert hat. Weiterhin enthält der von dem Analyseberichtgenerator 76 generierte Analysebericht eine Bewertung der jeweils detektierten Auffälligkeit in Form eines Hinweises, z.B. "Priorität hoch", "Priorität mittel" oder "Priorität niedrig". Schließlich enthält der Analysebericht eine jeweilige Handlungsempfehlung für den Arzt, wie sie durch die Empfehlungseinheit 74 generiert wurde.

Der so generierte Analysebericht steht schließlich an der Ausgangsschnittstelle 34 zur Weitergabe an ein Anzeigegerät, wie beispielsweise einen Bildschirm oder ein anderes Wiedergabegerät, wie z.B. einen Drucker, zur Verfügung. Dabei versteht es sich, dass dieses Wiedergabegerät auch weit entfernt von der Analyseeinheit 32 angeordnet sein kann, beispielsweise über das Internet mit der Analyseeinheit 32 verbunden ist.

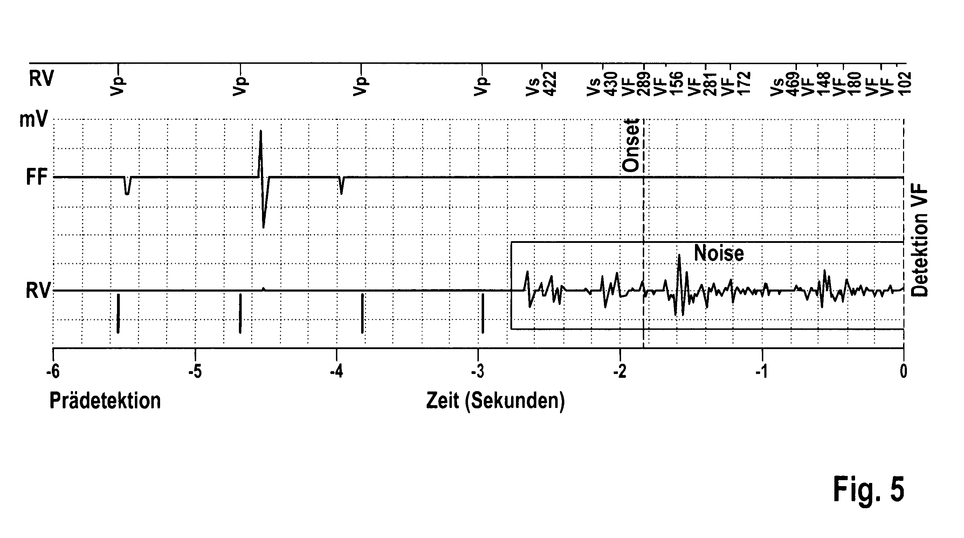

Die Figuren 5 bis 8 zeigen verschiedene IEGM Präsentationen in beispielhaften Analyseberichten, wie sie von dem Analyseberichtgenerator 76 auf Basis der Auswertung der Elektrokardiogrammsignale und Markersignale durch die Analyseeinheit 32 generiert wurden. Jeder Analysebericht enthält zunächst einen Hinweis auf die Priorität der detektierten Auffälligkeit, wie sie durch die Bewertungseinheit 72 festgelegt wurde. Darauf folgt eine Beschreibung der detektierten Auffälligkeit. Anschließend findet sich eine Repräsentation des jeweils betroffenen Signalabschnitts in grafischer Darstellung wie in den Figuren 5 bis 8 dargestellt. Schließlich enthält jeder Analysebericht auch noch eine Empfehlung für den jeweils behandelnden Arzt.

Für die in Figur 5 dargestellte IEGM Präsentation ist die Priorität "Hoch", die Beschreibung lautet:
"Nachweis von unregelmäßigen Rauschen im RV Kanal
   -> V.a. Elektrodenbruch"

Die Empfehlung ist:
"Durchführung von Reizschwellen-, Sensing- und Impedanzmessungen der RV-Elektrode, ggf. unter Provokation".

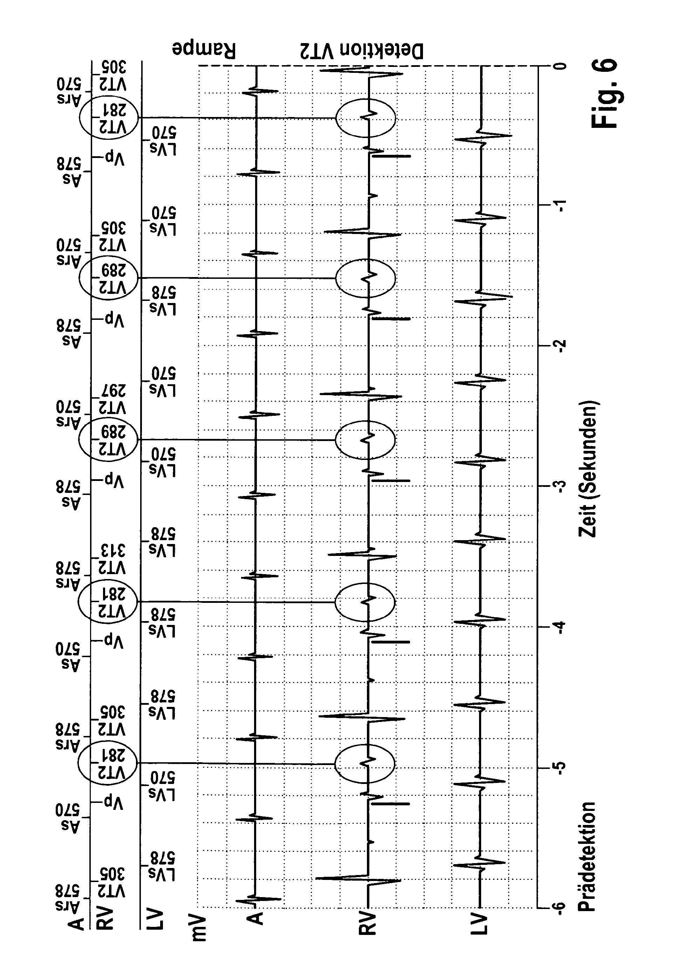

Für die in Figur 6 dargestellte IEGM Präsentation ist die Priorität "Hoch"; die Beschreibung lautet:
"Inadäquate VT-2 Detektion bei T-Wellen-Oversense im RV Kanal"

Die Empfehlung ist:
"Wenn VF-Detektion gewährleistet ist, Aktivierung der Anti-T-Wellen Oversense Option nach Pace".

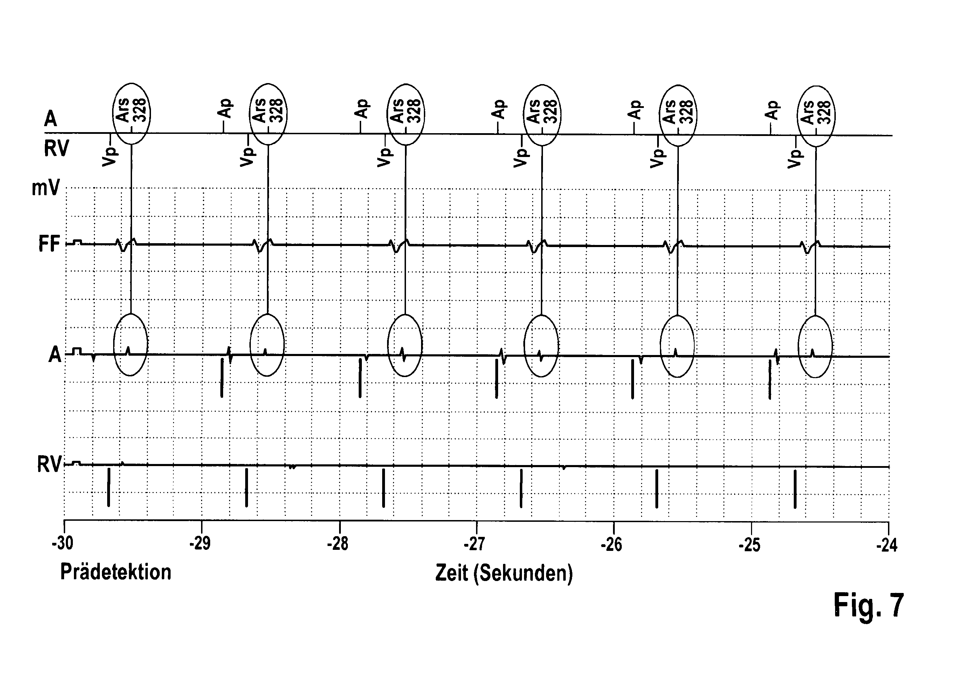

Für die in Figur 7 dargestellte IEGM Präsentation ist die Priorität "Mittel"; die Beschreibung lautet:
"Farfield-Sensing nach RV Stimulation im Atrialen Kanal.
   -> Dies kann zu inadäquaten Mode-Switch führen
   -> Dies kann falsch positive Ergebnisse in den Atrialen Arrhythmie-Statistiken führen"

Die Empfehlung ist:
"Überprüfung der RA-Sondenlage
   - ggf. Erweiterung des Cross-Channel-Blanks im Atrium um 30 ms.".

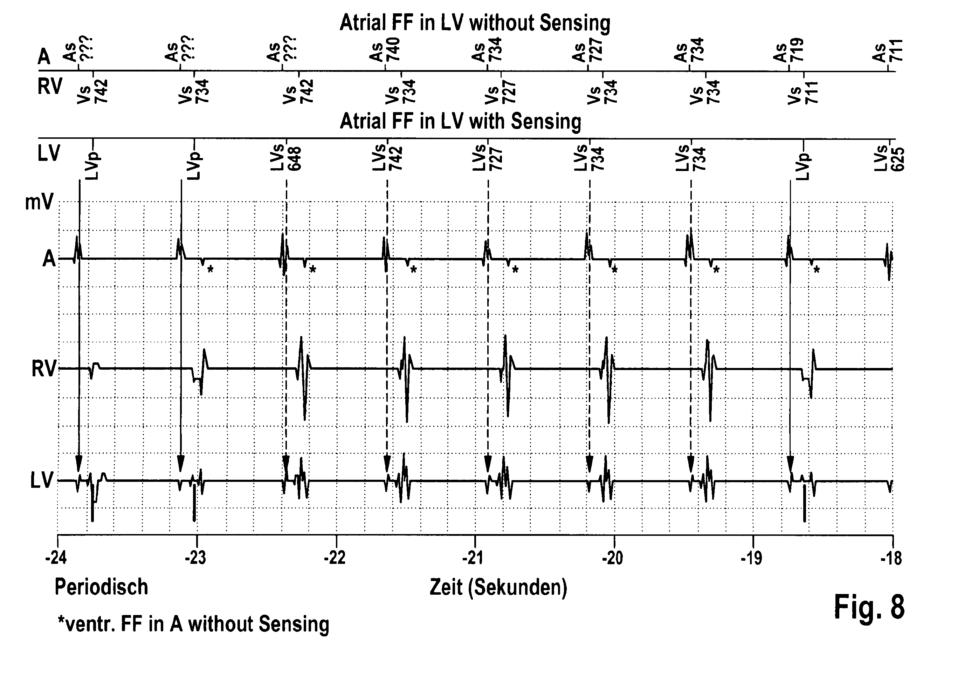

Für die in Figur 8 dargestellte IEGM Präsentation ist die Priorität "Mittel"; die Beschreibung lautet:
"Farfield-Sensing des Atrium im LV-Kanal
   -> Dies kann bei aktivierter LV-Protection zur inadäquaten Unterdrückung der LV Stimulation und damit zur Verschlechterung der CRT Therapie führen"

Die Empfehlung ist:
"- Überprüfung der LV-Sondenlage
   - ggf. ist eine Abschaltung der LV-T-Wellen Protektion notwendig oder Reduktion der Empfindlichkeit im LV-Kanal notwendig".

Auf diese Weise macht es die Analyseeinheit 12 dem Arzt wesentlich leichter, Auffälligkeiten zu detektieren und ggf. nötige Handlungen vorzunehmen.

## Patentansprüche

1. Vorrichtung (10) zur Verarbeitung von Elektrokardiogrammsignalen und Markersignalen, mit
- einer Eingangsschnittstelle (30) zum Empfang seitens eines implantierbaren medizinischen Gerätes erfasster Elektrokardiogrammsignale und Markersignale,
- einer mit der Eingangsschnittstelle (30) verbundenen Analyseeinheit (32) zum Verarbeiten der Elektrokardiogrammsignale, Marker- und Implantatdaten, und
- einer mit der Analyseeinheit (32) verbundenen Ausgangsschnittstelle (34) zur Ausgabe von einen Analysebericht repräsentierenden Daten an ein Wiedergabegerät,
wobei die Analyseeinheit (32) einen Ereignisdetektor (54) aufweist, der ausgebildet ist in einem Elektrokardiogrammsignal Abschnitte zu detektieren, die für eine Depolarisation des Myokards charakteristisch sind und somit kardiale Ereignisse repräsentieren und für diese Ereignisse Markersignale zu erzeugen,
wobei die Analyseeinheit (32) einen Markerkorrelator (56) aufweist, der ausgebildet ist, zeitliche Korrelationen zwischen den vom Ereignisdetektor (54) detektierten kardialen Ereignissen und entsprechenden Markern in dem über die Eingangschnittstelle (30) empfangenen Markersignal zu bestimmen und die in der Analyseeinheit generierten Markersignale mit den über die Eingangsschnittstelle empfangenen Markersignalen zu vergleichen, wobei die Analyseeinheit ausgebildet ist, die Elektrokardiogrammsignale und die über die Eingangsschnittstelle empfangenen Markersignale in einer Zusammenschau derart zu analysieren, dass die Analyseeinheit relevante Auffälligkeiten anhand vorgegebener Detektionskriterien detektiert, einer detektierten Auffälligkeit eine Priorität zuordnet, und einen Analysebericht generiert, der zumindest eine Auflistung der detektierten Auffälligkeiten enthält.

2. Vorrichtung nach Anspruch 1, **gekennzeichnet durch** eine Eingabeschnittstelle (40), die mit der Analyseeinheit (32) verbunden und in Verbindung mit dieser ausgebildet ist, Benutzvorgaben entgegenzunehmen, welche die Arbeitsweise der Analyseeinheit bei der Verarbeitung der Elektrokardiogrammsignale und Markersignale beeinflussen.

3. Vorrichtung nach Anspruch 1, **dadurch gekennzeichnet, dass** die Analyseeinheit einen Rauschdetektor (50) aufweist, der ausgebildet ist, in einem Elektrokardiogrammsignal Abschnitte zu detektieren, die über einen vorgegeben Grenzwert hinaus verrauscht sind.

4. Vorrichtung nach Anspruch 1, **dadurch gekennzeichnet, dass** die Analyseeinheit einen Artefaktdetektor (52) aufweist, der ausgebildet ist, in einem Elektrokardiogrammsignal Abschnitte zu detektieren, die Artefakte wie Polarisationsartefakte aufweisen.

5. Vorrichtung nach Anspruch 1, **dadurch gekennzeichnet, dass** die Analyseeinheit eine Oversensing-Klassifikationseinheit (66) aufweist, die ausgebildet ist, in einem Elektrokardiogrammsignal Abschnitte zu detektieren, die Signalmerkmale aufweisen, die auf Oversensing hinweisen.

6. Vorrichtung nach Anspruch 1, **dadurch gekennzeichnet, dass** der Ereignisdetektor (54) eine Morphologieklasssifikationseinheit (58) aufweist oder mit dieser verbunden ist, die ausgebildet ist, Signalabschnitte, die für eine Depolarisation des Myokards charakteristisch sind, auf dem Wege einer Signalformanalyse zu detektieren

7. Vorrichtung nach Anspruch 1, **gekennzeichnet durch** eine Bewertungseinheit (72), die mit der Analyseeinheit (12) verbunden und die ausgebildet ist, von der Analyseeinheit (12) detektierte Auffälligkeiten anhand vorgegebener Kriterien hinsichtlich ihrer Bedeutung derart zu bewerten, dass weniger bedeutende Auffälligkeiten eine andere Bewertung erfahren, als bedeutendere Auffälligkeiten.

8. Vorrichtung nach Anspruch 9, **dadurch gekennzeichnet, dass** die Analyseeinheit einen Berichtsgenerator (76) aufweist, der ausgebildet ist, einem Analysebericht jeweils eine von der Bewertungseinheit generierte Bewertung hinzuzufügen.

9. Vorrichtung nach Anspruch 1, **gekennzeichnet durch** eine Empfehlungseinheit (74), die mit der Analyseeinheit (12) verbunden und ausgebildet ist, auf Basis von der Analyseeinheit (12) detektierter Auffälligkeiten eine Therapieempfehlung zu generieren.

10. Vorrichtung nach einem der Ansprüche 1 bis 11, **dadurch gekennzeichnet, dass** die Auflistung der detektierten Auffälligkeiten durch eine Priorisierung und/oder eine Wiedergabe eines Elektrokardiogrammsignal- und/oder eines Markersignalabschnitts in graphischer Wiedergabe ggf. mit Markierung der die Auffälligkeit repräsentierenden Signalabschnitte und eine Benennung der Auffälligkeit ergänzt.

## Claims

1. A device (10) for processing electrocardiogram signals and marker signals, comprising
- an input interface (30) for receiving electrocardiogram signals and marker signals detected by an implantable medical device,
- an analysis unit (32) connected to the input interface (30) for processing the electrocardiogram signals, marker data and implant data, and
- an output interface (34) connected to the analysis unit (32) for outputting data representing an analysis report to a display device,
wherein the analysis unit (32) has an event detector (54), which is configured to detect portions in an electrocardiogram signal that are characteristic for a depolarisation of the myocardium and thus represent cardial events and to generate marker signals for these events,
wherein the analysis unit (32) has a marker correlator (56), which is configured to determine time correlations between the cardial events detected by the event detector (54) and corresponding markers in the marker signal received via the input interface (30) and to compare the marker signals generated in the analysis unit with the marker signals received via the input interface,
wherein the analysis unit is configured to analyse the electrocardiogram signals and the marker signals received via the input interface in an overview in such a way that the analysis unit detects relevant conspicuous features on the basis of predefined detection criteria, assigns a priority to a detected conspicuous feature and generates an analysis report, which contains at least a list of the detected conspicuous features.

2. The device according to Claim 1, **characterised by** an input interface (40), which is connected to the analysis unit (32) and is configured, in conjunction therewith, to accept use specifications that influence the functioning of the analysis unit when processing the electrocardiogram signals and marker signals.

3. The device according to Claim 1, **characterised in** the analysis unit has a noise detector (50), which is configured to detect portions in an electrocardiogram signal that are affected by noise beyond a predefined limit value.

4. The device according to Claim 1, **characterised in that** the analysis unit has an artefact detector (52), which is configured to detect portions in an electrocardiogram signal that have artefacts such as polarisation artefacts.

5. The device according to Claim 1, **characterised in that** the analysis unit has an oversensing classification unit (66), which is configured to detect portions in an electrocardiogram signal that have signal features indicating oversensing.

6. The device according to Clam 1, **characterised in that** the event detector (54) has a morphology classification unit (58) or is connected thereto, which morphology classification unit is configured to detect signal portions that are characteristic for a depolarisation of the myocardium by means of a signal form analysis.

7. The device according to Claim 1, **characterised by** an assessment unit (72), which is connected to the analysis unit (12) and which is configured to assess the significance of conspicuous features detected by the analysis unit (12) on the basis of predefined criteria, in such a way that less significant conspicuous features experience a different assessment compared with more significant conspicuous features.

8. The device according to Claim 9, **characterised in that** the analysis unit has a report generator (76), which is configured to add to an analysis report an assessment generated by the assessment unit.

9. The device according to Claim 1, **characterised by** a recommendation unit (74), which is connected to the analysis unit (12) and is configured to generate a therapy recommendation on the basis of conspicuous features detected by the analysis unit (12).

10. The device according to one of Claims 1 to 9, **characterised in that** the list of detected conspicuous features is supplemented by a prioritisation and/or a display of an electrocardiogram signal portion and/or a marker signal portion in graphical display, where appropriate with marking of the signal portions representing the conspicuous feature and a designation of the conspicuous feature.

## Revendications

1. Dispositif (10) pour le traitement de signaux d'électrocardiogramme et de signaux de marquage, avec
- une interface d'entrée (30) destinée à recevoir des signaux d'électrocardiogramme et des signaux de marquage détectés du côté d'un appareil médical implantable,
- une unité d'analyse (32) reliée à l'interface d'entrée (30) pour le traitement des signaux d'électrocardiogramme, des données de marquage et d'implant, et
- une interface de sortie (34) reliée à l'unité d'analyse (32) destinée à délivrer des données représentant un rapport d'analyse à un appareil de reproduction,
dans lequel l'unité d'analyse (32) comporte un détecteur d'évènements (54) conçu pour détecter, dans un signal d'électrocardiogramme, des sections caractéristiques d'une dépolarisation du myocarde, représentant ainsi des évènements cardiaques, et pour générer des signaux de marquage pour ces évènements,
dans lequel l'unité d'analyse (32) comporte un corrélateur de marquage (56) conçu pour déterminer des corrélations temporelles entre les évènements cardiaques détectés par le détecteur d'évènements (54) et des marqueurs correspondants dans le signal de marquage reçu par le biais de l'interface d'entrée (30), et pour comparer les signaux de marquage générés dans l'unité d'analyse avec les signaux de marquage reçus par le biais de l'interface d'entrée, sachant que l'unité d'analyse est conçue pour analyser dans une vue d'ensemble les signaux d'électrocardiogramme et les signaux de marquage reçus par le biais de l'interface d'entrée de manière à ce que l'unité d'analyse détecte les anomalies notables à l'aide de critères de détection prédéfinis, attribue une priorité à une anomalie détectée et génère un rapport d'analyse contenant au moins une liste d'anomalies détectées.

2. Dispositif selon la revendication 1, **caractérisé par** une interface de saisie (40) reliée à l'unité d'analyse (32) et conçu pour recevoir, conjointement avec celle-ci, des préférences d'utilisation influençant le travail de l'unité d'analyse lors du traitement des signaux d'électrocardiogramme et des signaux de marquage.

3. Dispositif selon la revendication 1, **caractérisé en ce que** l'unité d'analyse comporte un détecteur de bruit (50) conçu pour détecter, dans un signal d'électrocardiogramme, des sections dont le bruit dépasse une valeur seuil prédéfinie.

4. Dispositif selon la revendication 1, **caractérisé en ce que** l'unité d'analyse comporte un détecteur d'artefact (52) conçu pour détecter, dans un signal d'électrocardiogramme, des sections comportant des artefacts tels que des artefacts de polarisation.

5. Dispositif selon la revendication 1, **caractérisé en ce que** l'unité d'analyse comporte une unité de classification par sur-détection (66) conçue pour détecter, dans un signal d'électrocardiogramme, des sections comportant des caractéristiques de signal indiquant une sur-détection.

6. Dispositif selon la revendication 1, **caractérisé en ce que** le détecteur d'évènements (54) comporte une unité de classification par morphologie (58) ou est relié à celle-ci, laquelle est conçue pour détecter des sections de signal caractéristiques d'une dépolarisation du myocarde, à l'aide d'une analyse de forme de signal.

7. Dispositif selon la revendication 1, **caractérisé par** une unité d'appréciation (72) reliée à l'unité d'analyse (12) et conçue pour évaluer les anomalies détectées par l'unité d'analyse (12) quant à leur importance à l'aide de critères prédéfinis, de manière à attribuer aux anomalies moins importantes une autre appréciation qu'aux anomalies plus importantes.

8. Dispositif selon la revendication 9, **caractérisé en ce que** l'unité d'analyse comporte un générateur de rapport (76) conçu pour associer respectivement une appréciation générée par l'unité d'appréciation à un rapport d'analyse.

9. Dispositif selon la revendication 1, **caractérisé par** une unité de recommandation (74) reliée à l'unité d'analyse (12) et conçue pour générer une recommandation thérapeutique sur la base des anomalies détectées par l'unité d'analyse (12).

10. Dispositif selon l'une des revendications 1 à 9, **caractérisé en ce que** la liste des anomalies détectée est complétée par une priorisation et/ou une reproduction d'une section de signal d'électrocardiogramme et/ou de signal de marquage, dans une reproduction graphique, le cas échéant avec marquage des sections de signal représentant l'anomalie, et par une désignation de l'anomalie.
